(19) Europäisches Patentamt
European Patent Office
Office européen des brevets

(11) **EP 3 914 584 B1**

(12) **EUROPEAN PATENT SPECIFICATION**

(45) Date of publication and mention of the grant of the patent:
**12.10.2022 Bulletin 2022/41**

(21) Application number: **20757617.4**

(22) Date of filing: **24.08.2020**

(51) International Patent Classification (IPC):
**C07C 317/14** *(2006.01)*

(52) Cooperative Patent Classification (CPC):
**C07C 317/14**

(86) International application number:
**PCT/EP2020/073630**

(87) International publication number:
**WO 2021/037797 (04.03.2021 Gazette 2021/09)**

(54) **(C)CRYSTAL COMPOSITION (CC) COMPRISING 4,4'-DICHLORODIPHENYLSULFONE CRYSTALS (C)**

KRISTALLZUSAMMENSETZUNG MIT 4,4-DICHLORDIPHENYLSULFOXID-KRISTALLEN

COMPOSITION CRISTALLINE (CC) COMPRENANT DES CRISTAUX DE 4,4-DICHLORODIPHÉNYLSULFONE (C)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2019 EP 19193679**

(43) Date of publication of application:
**01.12.2021 Bulletin 2021/48**

(73) Proprietor: **BASF SE**
**67056 Ludwigshafen am Rhein (DE)**

(72) Inventors:
• **GAO, Jun**
**67056 Ludwigshafen (DE)**
• **THIEL, Indre**
**67056 Ludwigshafen (DE)**
• **HAMANN, Jessica Nadine**
**67056 Ludwigshafen (DE)**

• **THRUN, Frauke**
**67056 Ludwigshafen (DE)**
• **SCHUETZ, Christian**
**67056 Ludwigshafen (DE)**
• **BLEI, Stefan**
**67056 Ludwigshafen (DE)**

(74) Representative: **BASF IP Association**
**BASF SE**
**G-FLP-C006**
**67056 Ludwigshafen (DE)**

(56) References cited:
**WO-A1-2018/007481      CN-B- 106 588 719**
**US-A- 4 016 210**

Remarks:
The file contains technical information submitted after the application was filed and not included in this specification

**Description**

The invention relates to crystals (C) consisting of at least 98 % by weight of 4,4'-dichlorodiphenylsulfone, 0 to 2 % by weight of impurities and 0 to 2 % by weight of at least one solvent (c). Moreover, the present invention relates to a crystal composition (CC) comprising crystals (C) and a process for the production of the crystal composition (CC) and the crystals (C).

4,4'-dichlorodiphenylsulfone is also called 1,1'-sulfonylbis(4-chlorobenzene) or bis(4-chlorophenyl) sulfone. 4,4'-dichlorodiphenylsulfone is a white solid and has a molecular weight of 287.15 g/mol, a chemical formula $C_{12}H_8Cl_2O_2S$ and the CAS-registry-number of 4,4'-dichlorodiphenylsulfone is 80-07-9.

4,4'-dichlorodiphenylsulfone is commercially available, for example from Sigma Aldrich, Alfa Aesar and TCI.

4,4'-Dichlorodiphenylsulfone is a monomer which is used in polymerization processes for the production of polysulfones, polyethersulfones and polyphenylensulfones.

For the production of 4,4'-dichlorodiphenylsulfone several processes are known. In the so-called Rutherford Process 4,4'-dichlorodiphenylsulfone is produced by reacting chlorobenzene with sulfur trioxide ($SO_3$) and dimethylsulfate. In the so-called Amoco Process 4,4'-dichlorodiphenylsulfone chlorobenzene is reacted with $SO_3$ at 80 °C to form 4-chlorobenzenesulfonic acid which is reacted at 220°C with further chlorobenzene to form 4,4'-dichlorodiphenylsulfone.

The synthesis of 4,4'-dichlorodiphenylsulfone can also be carried out in a two step process.

In the first step 4,4'-dichlorodiphenylsulfoxide is produced. For the production of 4,4'-dichlorodiphenylsulfoxide several processes are known. One common process is a *Friedel-Crafts-Reaction* with thionyl chloride and chlorobenzene as starting materials in the presence of a catalyst, for example aluminum(III)chloride or iron(III)chloride. Sun, X. et al, "Iron(III) chloride (FeCl3)-catalyzed electrophilic aromatic substitution of chlorobenzene with thionyl chloride (SOCl2) and the accompanying auto-redox in sulfur to give diaryl sulfides (Ar2S): Comparison to catalysis by aluminum chloride (AlCl3)", phosphorus, sulfur, and silicon, 2017, Vol. 192, No. 3, pages 376 to 380, and Sun, X. et al, "Investigations on the Lewis-acids-catalysed electrophilic aromatic substitution reactions of thionyl chloride and selenyl chloride, the substituent effects, and the reaction mechanisms", Journal of Chemical Research 2013, pages 736 to 744, discloses general processes for the production of 4,4'-dichlorodiphenylsulfoxide.

In the second step the 4,4'-dichlorodiphenylsulfoxide is oxidized with peroxide in the presence of an acid solvent to obtain4,4'-dichlorodiphenylsulfone. As a peroxide a organic peracid or a mixture of hydro peroxide and a organic acid like a carboyxylic acid is use. A preferred peroxide is heptanoic peracid. A suitable process for the oxidization of 4,4'-dichlorodiphenylsulfoxide to 4,4'-dichlorodiphenylsulfone is described in the international application WO 2018/007481.

CN 106588719 discloses a process for the purification of 4,4'-dichlorodiphenylsulfone, wherein the 4,4'-dichlorodiphenylsulfone is dissolved in toluene and subsequently treated with sodium hydroxide, EDTA and activated carbon, followed by filtration and recrystallization. However, the 4,4'-dichlorodiphenylsulfone obtained by this process still contains a high amount of impurities.

Commercially available 4,4'-dichlorodiphenylsulfone is provided in particulate powder form or in crystalline powder form. In the processes described in the above mentioned documents 4,4'-dichlorodiphenylsulfone is also obtained in particulate powder form or in crystalline powder form.

The powdery 4,4'-dichlorodiphenylsulfones commercially available, and the powdery 4,4'-dichlorodiphenylsulfones obtained in the processes described in the above mentioned document, however, for some applications show insufficient flowability. Likewise, the known powdery 4,4'-dichlorodiphenylsulfones show quite high bulk densities as well as quite high tapered densities, which can lead to storage problems like caking. Moreover, in some cases the content of by-product (impurities) and the content of residual organic solvents contained in the known 4,4'-dichlorodiphenylsulfone is too high.

Therefore, the object underlying the present invention is to provide 4,4'-dichlorodiphenylsulfone in particulate form, which does not have the above-mentioned disadvantages of the prior art or has them only in a significantly reduced extent.

This object was solved by a crystal composition (CC) comprising crystals (C), wherein the crystals (C) consist of

(a) at least 99.95 % by weight of 4,4'-dichlorodiphenylsulfone,

(b) 0 to 0.05 % by weight of impurities, and

(c) 0 to 0.05 % by weight of at least one solvent,

based on the total weight of the crystals (C) contained in the crystal composition (CC), wherein the crystal composition (CC) has a bulk density determined according to EN ISO 60:2000-01 in the range of 570 to 750 kg/m³.

The content of 4,4'-dichlorodiphenylsulfon, impurities and the at least one solvent of the crystals (C) are determined as described in the examples.

It has been found that, surprisingly, the inventive crystal composition (CC) shows a better flowability compared to the particulate 4,4'-dichlorodiphenylsulfones described in the state of the art. Moreover, it has been found, surprisingly, that the inventive crystal composition (CC) has a lower bulk density as well as a lower tapered density which leads to an improved storability. Likewise, it has been found that the crystals (C) comprised in the crystal composition (CC) have a low content of by-products (impurities), a low content of residual solvent(s) as well as a low APHA-color number.

*Crystal composition (CC)*

The crystal composition (CC) comprises crystals (C). In a preferred embodiment the crystal composition (CC) comprises at least 95 % by weight of the crystals (C), more preferred the crystal composition (CC) comprises at least 98 % by weight of crystals (C) even more preferred the crystal composition (CC) comprises at least 99 % by weight of the crystals (C) and particularly preferred the crystal composition (CC) comprises at least 99,5 % by weight of crystals (C) in each case based on the total weight of the crystal composition (CC). In an even more preferred embodiment, the crystal composition (CC) consists of the crystals (C).

Therefore, another object of the present invention is a crystal composition (CC), wherein the crystal composition (CC) comprises at least 95 % by weight of crystals (C), based on the total weight of the crystal composition (CC).

The crystal composition (CC) of the invention generally has:

a $d10x_{c\,min}$-value in the range of 30 to 120 $\mu$m,
a $d50x_{c\,min}$-value in the range of 150 to 350 $\mu$m and
a $d90x_{c\,min}$-value in the range of 300 to 600 $\mu$m.

Preferably, the crystal composition (CC) of the invention has:

a $d10x_{c\,min}$-value in the range of 35 to 60 $\mu$m,
a $d50x_{c\,min}$-value in the range of 170 to 300 $\mu$m and
a $d90x_{c\,min}$-value in the range of 300 to 500 $\mu$m.

In each case on condition that the $d10x_{c\,min}$-value is lower than the $d50x_{c\,min}$-value and the $d50x_{c\,min}$-value is lower than the $d90x_{c\,min}$-value.

In the context of the present invention the "$d10x_{c\,min}$-value", "$d50x_{c\,min}$-value", and "$d10x_{c\,min}$-value" describe the particle sizes based on the volume of the particles.

In the context of the present invention, the "$d10x_{c\,min}$-value" is understood to mean the particle size at which 10% by volume of the particles, preferably the crystals (C), based on the total volume of the particles, preferably the crystals (C), are smaller than or equal to the $d10x_{c\,min}$-value and 90% by volume of the particles, preferably the crystals (C), based on the total volume of the particles, preferably the crystals (C), are larger than the $d10x_{c\,min}$-value. By analogy, "$d50x_{c\,min}$-value" is understood to mean the particle size at which 50% by volume of the particles, preferably the crystals (C), based on the total volume of the particles, preferably the crystals (C), are smaller than or equal to the $d50x_{c\,min}$-value and 50% by volume of the particles, preferably the crystals (C), based on the total volume of the particles, preferably the crystals (C), are larger than the $d50x_{c\,min}$-value. Correspondingly, the "$d90x_{c\,min}$-value" is understood to mean the particle size at which 90% by volume of the particles, preferably the crystals (C), based on the total volume of the particles, preferably the crystals (C), are smaller than or equal to $d90x_{c\,min}$-value and 10% by volume of the particles, preferably the crystals (C), based on the total volume of the particles, preferably the crystals (C), are larger than $d90x_{c\,min}$-value.

The particle sizes of the crystals (C) comprised in the crystal composition (CC), the $d10x_{c\,min}$-values, the $d50x_{c\,min}$-values and the $d90x_{c\,min}$-values, as well as the average aspect ratios $(b/l_3)$, the average sphericity $(SPTH_3)$, the average $X_{c\,min}$ diameter and the average maximum Feret diameter $(X_{Fe\,max})$ are determined with a Camsizer® XT (of the company Retsch Technology) using the measuring methods described in the manual "CAMSIZER® Characteristics, Basics of definition DIN 66141, Retsch Technology dated November 5, 2009" which is available under the following www.-link: http://www.horiba.com/fileadmin/uploads/Scientific/Documents/PSA/Manuals/CAMSIZER_Characteristics_Nov2009.pdf

The particle sizes (hereinafter the wording "particle size" and "particle diameter" are used synonymously and have the same meaning) are determined on basis of definition DIN 66141 dated February 1974. Therefore, the crystal composition (CC) is fed via a vibrating feeder past the measurement optic of the Camsizer® XT at room temperature (20°C) and normal pressure (1,01325 bar), wherein at least 80 000 particles, preferably crystals (C), are measured.

The $d10_{,3}$-values, the $d50_{,3}$-values and the $d90_{,3}$-values are determined by the $X_{area}$ method. With the measuring method $X_{area}$ the particle diameter is calculated by the area of particle projection using the following formula:

$$X_{area} = \sqrt{\frac{4A}{\pi}}$$

wherein the diameter of the area equivalent circle with a volume of a sphere with the diameter of $X_{area}$ is determined.

The bulk density of the crystal composition (CC) is generally in the range of 570 to 750 kg/m$^3$, preferably in the range of 600 to 720 kg/m$^3$ and more preferably in the range of 650 to 710 kg/m$^3$. The bulk density of the crystal composition (CC) is determined according to EN ISO 60:2000-01.

The tapered density (measured after 1250 lifts) of the crystal composition (CC) is generally in the range of 750 to 850 kg/m$^3$ and preferably in the range of 700 to 900 kg/m$^3$. The tapered density of the crystal composition (CC) is determined according to DIN ISO 787 part 11 (after 1250 lifts).

The Hausner ratio of the crystal composition (CC) is generally in the range of 1.05 to 1.25, preferably in the range of 1.1 to 1.2 and more preferably in the range of 1.14 to 1.18.

The Hausner ratio is the ratio of tapered density (preferably after 1250 lifts) to bulk density. The Hausner ratio is a parameter for the flowability of particulate compositions, wherein the flowability is classified according to the following table:

| Hausner ratio | Flowability |
| --- | --- |
| 1.05-1.18 | Excellent |
| 1.14-1.19 | Good |
| 1.22 - 1.27 | Acceptable |
| 1.3 - 1.54 | Poor |
| 1.49 -1.61 | Very Poor |
| > 1.67 | Not Flowing |

Another object of the present invention, therefore, is a crystal composition (CC), wherein the Hausner ratio is in the range of 1.05 to 1.25.

The crystal composition (CC) preferably has a flowability (ff$_c$) according to Jenike and ASTM D7891 - 15 at an initial shear stress of 3kPa in the range of 10 to 50, preferably in the range of 15 to 35, more preferably in the range of 18 to 30 and particularly preferred in the range of 20 to 26. The flowability (ff$_c$) is determined on a Freeman FT4 according to Jenike as described in ASTM D7891 - 15 "Standard Test Method for Shear Testing of Powders Using the Freeman Technology FT4 Powder Rheometer Shear Cell" at an initial shear stress of 9 kPa.

Another object of the present invention, therefore, is a crystal composition (CC), wherein the flowability (ff$_c$) according to Jenike of the crystal composition (CC) is in the range of 10 to 50.

According to Jenike the flowability is classified according the following table:

| ff$_c$ | Flowability |
| --- | --- |
| < 1 | Not flowing |
| 1 < to < 2 | Very poor |
| 2 < to < 4 | Poor |
| 4 < to < 10 | Good |
| 10 < | Excellent |

The crystals (C) contained in the crystal composition (CC) according to the invention generally have an average aspect ratio in the range of 0.2 to 1, preferably in the range of 0.4 to 0.8 and more preferably in the range of 0.55 to 0.7.

Another object of the present invention, therefore, is a crystal composition (CC), wherein the average aspect ratio of the crystals (C) contained in the crystal composition (CC) is in the range of 0.2 to 1.

The average aspect ratio of the crystals (C) comprised in the crystal composition (CC) is determined with a Camsizer® XT using the method b/l$_3$ as described in the above referenced manual on basis of definition DIN 66141 dated February 1974. The aspect ratio is calculated by using the following formula:

$$b/l_3 = \frac{X_{c\ min}}{X_{Fe\ max}}$$

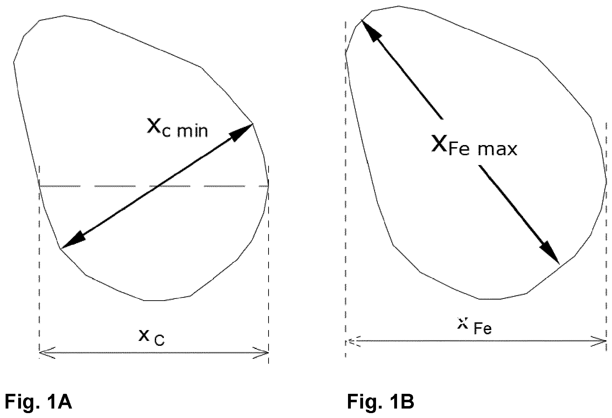

$X_{c\ min}$ is the volume average particle diameter which is the shortest cort of the measured set of maximum corts of the particle projection (the crystal (C) projection). Figures 1A and 1B shows an example, how $X_{c\ min}$ is measured. $X_{c\ min}$ is the volume average of the shortest cort over all particles (crystals (C)), comprised in the crystal composition (CC).

The maximum feret diameter ($X_{Fe\ max}$) is the volume average particle diameter over all particles (crystals (C)), comprised in the crystal composition (CC), which is the longest ferret diameter of the measured set of feret diameter of a particle. The determination of the maximum feret diameter $x_{Fe\ max}$ is shown by the way of example in figures 1A and 1B.

The crystals (C) contained in the crystal composition (CC) according to the invention have generally an average sphericity ($SPHT_3$) in the range of 0.6 to 0.9 and preferably in the range of 0.7 to 0.85. The sphericity is measured according to ISO 9276-6:2012-1.

Therefore, another object of the present invention is a crystal composition (CC), wherein the average sphericity of the crystals (C) is in the range of 0.6 to 0.9.

The crystal composition (CC) has generally an APHA-color number (ASTM D1209) in the range of 0 to 50, preferably in the range of 5 to 40, more preferably in the range of 10 to 30. The APHA-color numbers were measured on a Hach Lange LICO 500 instrument; 2.5 g 4,4'-DCDPS (4,4'-DCDPS = 4,4'dichlorodiphenylsulfone) were dissolved in 20 ml NMP and measured against pure NMP (NMP = N-Methyl-2-pyrrolidone).

Another object of the present invention is a crystal composition (CC) which has an APHA-color number determined according to ASTM D1209 in the range of 0 to 50.

*Crystals (C)*

The crystal (C) can differ from the crystals (C) comprised in the crystal composition (CC). In a preferred embodiment, the crystal (C) does not differ from the crystals (C) comprised in the crystal composition (CC). In a preferred embodiment, therefore, the features and preferences mentioned above in a view of the crystal composition (CC) apply for the crystal (C) accordingly. In another preferred embodiment, therefore, the features and preferences mentioned hereinafter in view of the crystal (C) apply for the crystal composition (CC) accordingly.

In a preferred embodiment the crystals (C) comprise at least 99.96% by weight, more preferably at least 99.97% by weight and most preferably at least 99.975% by weight of 4,4'-dichlorodiphenylsulfone, based in each case on the total weight of the crystals (C). The components (a), (b) and (c) comprised in the crystals (C) in a preferred embodiment ad up to 100% by weight. In case the crystals (C) do not comprise impurities (b) and solvents (c) the crystals consist of 100% of 4,4'-dichlorodiphenylsulfone.

In a preferred embodiment the crystals (C) comprise from 0 to 0.04% by weight, more preferably from 0 to 0.03% by weight and most preferably 0.025% by weight of impurities (b), based in each case on the total weight of the crystals (C).

In a preferred embodiment the crystals (C) comprises from 0 to 0.04% by weight, more preferably from 0 to 0.03% by weight, and most preferably from 0 to 00.025% by weight of at least one solvent (c), based in each case on the total weight of the crystals (C).

Another object of the present invention are crystals (C) wherein the impurities (b) comprise at least 90 % by weight, preferably at least 95 % by weight, more preferably at least 98 % by weight and particularly preferred at least 99 % by weight of one or more compounds selected from the group consisting of 2,4'-dichlorodiphenylsulfone, 3,4'-dichlorodiphenylsulfone, 4,4'-dichlorodiphenylsulfoxide, 2,4'- dichlorodiphenylsulfoxide and one or more carboxylic acid compound(s), in each case based on the total weight of the impurities (b) contained in the crystals (C).

In another particularly preferred embodiment the impurities (b) contained in the crystals (C) consist of one or more compounds selected from the group consisting of 2,4'-dichlorodiphenylsulfone, 3,4'-dichlorodiphenylsulfone, 4,4'-dichlorodiphenylsulfoxide, 2,4'-dichlorodiphenylsulfoxide and one or more carboxylic acid compound(s).

The carboxylic acid compound(s) optionally contained as impurities (b) in the crystals (C) may be be only one carboxylic acid or a mixture of at least two different carboxylic acids. Preferably the carboxylic acid is at least one aliphatic carboxylic acid. The at least one aliphatic carboxylic acid may be at least one linear or at least one branched aliphatic carboxylic acid or it may be a mixture of one or more linear and one or more branched aliphatic carboxylic acids. Preferably the aliphatic carboxylic acid is an aliphatic $C_6$ to $C_{10}$ carboxylic acid, particularly a $C_6$ to $C_9$ carboxylic acid, whereby it is particularly preferred that the at least one carboxylic acid is an aliphatic monocarboxylic acid. Thus, the at least one carboxylic acid may be hexanoic acid, heptanoic acid, octanoic acid nonanoic acid or decanoic acid or a mixture of one or more of said acids. For instance the at least one carboxylic acid may be n-hexanoic acid, 2-methyl-pentanoic acid, 3-methyl-pentanoic acid, 4-methyl-pentanoic acid, n-heptanoic acid, 2-methyl-hexanoic acid, 3-methyl-hexanoic acid, 4-methyl-hexanoic acid, 5-methyl-hexanoic acid, 2-ethyl-pentanoic acid, 3-ethyl-pentanoic acid, n-octanoic acid, 2-methyl-heptanoic acid, 3-methyl-heptanoic acid, 4-methyl-heptanoic acid, 5-methyl-heptanoic acid, 6-methyl-heptanoic acid, 2-ethyl-hexanoic acid, 4-ethyl-hexanoic acid, 2-propyl pentanoic acid, 2,5-dimethylhexanoic acid, 5,5-dimethyl-hexanoic acid, n-nonanoic acid, 2-ethyl-hepatnoic acid, n-decanoic acid, 2-ethyl-octanoic acid, 3-ethyl-ocantoic acid, 4-ethyl-octanoic acid. The carboxylic acid may also be a mixture of different structural isomers of one of said acids. For instance, the at least one carboxylic acid may be isononanoic acid comprising a mixture of 3,3,5-trimethyl-hexanoic acid, 2,5,5-trimethyl-hexanoic acid and 7-methyl-octanoic acid or neodecanoic acid comprising a mixture of 7,7-dimethyloctanoic acid, 2,2,3,5-tetramethyl-hexanoic acid, 2,4-dimethyl-2-isopropylpentanoic acid and 2,5-dimethyl-2-ethylhexanoic acid. Particularly preferably, however the carboxylic acid is n-hexanoic acid or n-heptanoic acid, wherein n-heptanoic acid is most preferred.

The content of the carboxylic acid compound(s) in the crystals (C) is preferably in the range of 0 to 200 ppm by weight, more preferably in the range of 0 to 150 ppm by weight and most preferably in the range of 0 to 100 ppm by weight, in each case based on the total weight of the crystals (C). The content of the carboxylic acid compound is determined as described below in the section examples.

The overall content of the isomers 2,4'-dichlorodiphenylsulfone, 3,4'-dichlorodiphenylsulfone, in the crystals (C) is preferably in the range of 0 to 300 ppm by weight, more preferably in the range of 0 to 200 ppm by weight and most preferably in the range of 0 to 100 ppm by weight, in each case based on the total weight of the crystals (C). The content of the above mentioned isomers is determined as described below in the section examples.

The overall content of 4,4'-dichlorodiphenylsulfoxide and 2,4'-dichlorodiphenylsulfoxide in the crystals (C) is preferably in the range of 0 to 50 ppm by weight, more preferably in the range of 0 to 20 ppm by weight and most preferably in the range of 0 to 10 ppm by weight, in each case based on the total weight of the crystals (C). The content of 4,4'-dichlorodiphenylsulfoxide is determined as described below in the section examples.

The crystals (C) comprise at least one solvent (c). In the context of the present invention the term "at least one solvent (c)" means exactly one solvent (c) as well as a mixture of two or more solvents (c).

The at least one solvent (c) may for example be water, a symmetric or asymmetric, branched or linear ethers, for example diethyl ether or methyl tert-butyl ether, substituted or unsubstituted aromatic solvents like toluene, monochlorobenzene or benzene, low molecular carboxylic acids, particularly $C_1$ to $C_3$ carboxylic acids or low molecular alcohols, particularly $C_1$ to $C_3$ alcohols. Preferably, the organic solvent is methanol, ethanol, isopropanol, acetone, methyl tert-butyl ether, acetic acid, toluene, ethyl acetate or monochlorobenzene. Particularly preferably, the organic solvent is a $C_1$ to $C_3$ alcohol, particularly methanol, ethanol or isopropanol. Most preferred the organic solvent is methanol.

Another preferred object of the present invention are crystals (C) wherein the solvent (c) comprises at least 98 % by weight of at least one solvent selected form the group consisting water diethyl ether, methyl tert-butyl ether, toluene, monochlorobenzene, and $C_1$ to $C_3$ alcohols, based on the total weight of the crystals (C).

Preferably the crystal (C) comprise at least 98 % of at least one solvent selected from the group consisting of water methanol, ethanol, isopropanol, acetone, methyl tert-butyl ether, acetic acid, toluene, ethyl acetate or monochlorobenzene based on the total weight of the crystals (C). Particularly preferably, the organic solvent is a water methanol, ethanol, isopropanol, toluene and/or monochlorobenzene. Most preferred the organic solvent is methanol.

The content of monochlorobenzene in the crystals (C) is preferably in the range of 0 to 50 ppm by weight, more preferably in the range of 0 to 20 ppm by weight and most preferably in the range of 0 to 10 ppm by weight, in each case based on the total weight of the crystals (C). The content of monochlorobenzene is determined as described below in the section examples.

The content of toluene in the crystals (C) is preferably in the range of 0 to 50 ppm by weight, more preferably in the range of 0 to 20 ppm by weight and most preferably in the range of 0 to 10 ppm by weight, in each case based on the total weight of the crystals (C). The content of toluene is determined as described below in the section examples.

The content of water in the crystals (C) is preferably in the range of 0 to 500 ppm by weight, more preferably in the range of 0 to 200 ppm by weight and most preferably in the range of 0 to 100 ppm by weight, in each case based on the total weight of the crystals (C). The content of water is determined as described below in the section examples.

To prepare the crystal composition (CC)/the crystals (C), in a preferred embodiment 4,4'-dichlorodiphenylsulfone is dissolved in the above mentioned at least one solvent (c) to obtain a solution of the 4,4'-dichlorodiphenylsulfone in the at least one solvent (c). Subsequently, the 4,4'-dichlorodiphenylsulfone is crystallized from the solution to obtain the crystal composition (CC)/the crystals (C). The crystallization can be carried out by all known methods like temperature reduction, removal of the solvent (c) etc.. For the crystallization of the 4,4'-dichlorodiphenylsulfone methanol is preferred as an solvent (c).

The invention and a method for the production of the crystal composition (CC)/the crystals (C) is described in more detail by the examples hereinafter without being restricted thereto.

*Examples*

1. Inventiv example; production of the crystal composition (CC)/the crystals (C) according to the invention

*Step 1: Production of 4,4'-dichlorodiphenyl sulfoxide (DCDPSO)*

5.5 mol aluminum chloride and 40 mol monochlorobenzene were fed into a stirred tank reactor as first reactor. 5 mol thionyl chloride were added to the reaction mixture in 160 min. The reaction in a first reactor was carried out at 10°C. Hydrogen chloride produced in the reaction was withdrawn from the process. After finishing the addition of thionyl chloride the reaction mixture was heated to 60°C.

After finishing the reaction in the first reactor the resulting reaction mixture was fed into a second stirred tank reactor which contained 3400 g hydrochloric acid with a concentration of 11 wt-%. The second stirred tank reactor was heated to a temperature of 90°C. After 30 min the mixing was stopped and the mixture separated into an aqueous phase and an organic phase.

The aqueous phase was withdrawn and the organic phase was washed with 3000 g water while stirring at 90°C. After washing, stirring was stopped and the mixture separated into an aqueous phase and an organic phase.

The aqueous phase was removed and the organic phase was subjected to a distillation. Monochlorobenzene was distilled from the organic phase until saturation was reached at about 88°C (monitored via a turbidity probe, distillation conditions: 200 mbar(abs)). The organic phase was cooled by reducing the pressure until the temperature reached 30°C.

By the cooling a suspension was obtained containing crystallized DCDPSO. The suspension then was filtrated to obtain a filter cake comprising crystallized DCDPSO, which was washed with 550 g monochlorobenzene.

The combined mother liquor and the monochlorobenzene which was used for washing were subjected to a distillation. In the distillation monochlorobenzene was removed until the amount of combined mother liquor and washing filtrate was reduced to 25 wt%. The distillation was operated at a bottom temperature of 90°C and 200 mbar(abs).

While the distilled monochlorobenzene was reused in the next batch as starting material, 80 wt% of the obtained bottom product were transferred into the crystallization of the next batch.

After washing with monochlorobenzene, the thus obtained monochlorobenzene-wet filter cake comprising crystallized DCDPSO was washed with 300 g n-heptanoic acid and filtrated to obtain n-heptanoic acid wet DCDPSO as filter cake.

The filtrate was subjected to distillation yielding a top fraction of monochlorobenzene and a bottom fraction containing n-heptanoic acid and DCDPSO. The bottom fraction was topped up with fresh n-heptanoic acid and reused in the next filtration. The distillation was operated at a bottom temperature of 140°C and 100 mbar(abs).

The 4,4'-dichlorodiphenyl sulfoxide yield in the steady state was 1232 g which corresponds to a yield of 91.3%.

The n-heptanoic acid wet DCDPSO had a purity of 89.7 wt%, containing 8.9 wt% n-heptanoic acid, 0.8 wt% monochlorobenzene, 0.3 wt% 4,4'-dichlorodiphenylsulfide and 0.3 wt% 2,4'-dichlorodiphenylsulfoxide.

*Step 2: Production of 4,4'-dichlorodiphenyl sulfone (DCDPS)*

1113 g of the n-heptanoic acid wet 4,4'-dichlorodiphenyl sulfoxide obtained in step 1 were dissolved in 2900 g n-heptanoic acid and heated to 90°C. 7.2 g sulfuric acid were added to the solution. Over a period of 3 h and 10 min 143 ml $H_2O_2$ were added to the solution with a constant feed rate. During the reaction the temperature in the vessel was controlled to 90°C by wall cooling, whereby the temperature in the reactor was determined to be 97 to 99°C. After finishing this step, the reactor was stirred for 15 minutes at a temperature of 97°C. Then, a second amount of 7 ml $H_2O_2$ was added within 10 minutes. After completing the $H_2O_2$ dosage the temperature of the solution was raised to 100°C. The reactor was stirred for 20 minutes at a temperature of 100°C.

To the resulting reaction mixture comprising DCDPS and n-heptanoic acid, 881 g water were added with a temperature of 97°C. The thus obtained mixture was cooled by reducing the pressure according to the cooling profile shown in table 1.

Table 1: cooling profile

| time [h] | temperature [°C] | pressure [mbar] |
| --- | --- | --- |
| 0:00 | 97 | 760 |
| 0:50 | 81 | 380 |
| 01:15 | 90 | 580 |
| 1:45 | 90 | 580 |
| 2:45 | 81 | 370 |
| 3:40 | 61,5 | 175 |

(continued)

| time [h] | temperature [°C] | pressure [mbar] |
|---|---|---|
| 4:35 | 43 | 70 |
| 6:00 | 18 | 980 |

A suspension comprising 2480 g n-heptanoic acid and DCDPS was obtained by this process.

The suspension then was filtered at ambient temperature to obtain a filter cake comprising about 80 wt% DCDPS, 16 wt% n-heptanoic acid and 4 wt% water. The mother liquor which was separated off the filter cake in the filtration process contained about 78 wt% n-heptanoic acid, 20 wt% water and about 2,5 wt% DCDPS. For filtering the suspension, a glass nutsche was used which was covered with a Sefar® Tetex DLW 17-80000-SK 020 Pharma filter cloth. For filtering, an absolute pressure of 500 mbar was set below the nutsche. After filtration, the filter cake was treated with dry air for 30 s.

*Step 3: washing the DCDPS with an aqueous base and water*

The filter cake obtained in step 2 then was washed with 2 kg of diluted NaOH 5%. For washing a pressure of 750 mbar(abs) were set to the filtrate side of the nutsche.

Washing with diluted NaOH was followed by washing with 1,5 kg water. For washing with water a pressure of 500 mbar(abs) were set to the filtrate side of the nutsche. Subsequently the filter cake was treated for 30 seconds with dried air.

After washing and treating with dried air, the filter cake contained about 20 wt% water and 0.24 wt% n-heptanoic acid. The final filter cake mass was 1369 g.

The mother liquor obtained in the filtration process was subjected to a phase separation. By phase separation, 482 g aqueous phase and 2712 g organic phase were obtained.

*Step 4:Re-crystallization of the DCDPS to obtain the crystal composition (CC)/the crystals (C)*

500.4 g of the filter cake obtained in step 3 containing 115 g water and containing about 0.24% n-heptanoic acid and about 240 ppm isomers of 4,4'-DCDPS were suspended into 1385 g methanol. This mixture was heated to a temperature of 100°C in a closed vessel. The temperature was kept at 100°C for 2h and 20 min. Then the pressure in the vessel was reduced and methanol started to evaporate. Evaporation of methanol resulted in crystallization of the DCDPS (crystals (C)). The temperature in the vessel was reduced linearly with a rate of 10 Kelvin per hour until a temperature of 10°C was reached. After this temperature was reached, the vessel was vented until ambient pressure was achieved. The thus obtained mixture of crystallized DCDPS (crystals (C)) and methanol was filtered in a filter nutsche. By this filtration a wet filter cake which weighted 613,5g was obtained. The wet filter cake was washed with 400g fresh methanol. Afterwards, the washed wet filter cake was dried for 5 hours in a Rotavapor® rotary evaporator with a wall temperature of 130°C. The thus obtained product (crystal composition (CC)) had given in the below table 2.

The particle analysis of the crystal composition (CC) obtained in step 4 gives the following result:

| | |
|---|---|
| $d10x_{c\,min}$-value: | 46 $\mu$m, |
| $d50x_{c\,min}$-value: | 181 $\mu$m, |
| $d90x_{c\,min}$-value: | 354 $\mu$m |
| Sphericity (Spht3): | 0.822 |
| Aspect ratio (b/l3): | 0.636 |

The crystal composition (CC) obtained in step 4 had a bulk density of 706 kg/m$^3$, a tapered density (1250 lifts) of 819 kg/m$^3$, a Hausner ratio of 1.16, a flowability according to Jenike of 24 and an APHA number of 24.

*Analytical methods*

The $d10x_{c\,min}$-values, the $d50x_{c\,min}$-values and the $d90x_{c\,min}$-values, sphericity (Spht3) and aspect ratio (b/l3) are determined as described above using a Camsizer®.

GC analysis was performed to determine any impurity (DCDPS Isomers, DCDPSO, monochlorobenzene, water), solvent (Methanol) and the purity of the 4,4'-dichlorodiphenylsulfone. Samples were diluted in dimethylformamide (DMF) and the internal standard tridecane was added to quantify the components based on calibration curves. GC analysis was performed using a RTx5 Amine column (0.25 $\mu$m) from Restek® using the following temperature ramp:

holding 50 °C for 2 minutes, heating 15 °C per minute until 250 °C is reached, holding 250 °C for 15 minutes. The column has a length of 30 m, an internal diameter of 250 $\mu$m and a film thickness of 0.25 $\mu$m. Helium is used as carrier gas with 1 ml/min (constant flow). The split ratio is 200:1. The injection and detector temperature are 300°C. The injection volume is 1 $\mu$l.

APHA numbers were measured (as described above) on a Hach Lange LICO 500 instrument; 2.5 g 4,4'-dichlorodiphenylsulfone were dissolved in 20 mL *N*-methyl-2-pyrrolidone (NMP) and measured against pure NMP.

The flowability according to Jenike, the Hausner ratio, the bulk density and the tapered density (1250 lifst) were determined as described above.

2. Storage tests

Storage tests were conducted at 25°C and 50% relative humidity (condition i) and at 40°C und 90% relative humidity (condition ii). The crystal composition (CC) of the inventive example was stored under the above mentioned conditions. The sample was examined after 2 and 4 weeks.

After 2 weeks and after 4 weeks of storage the sample of the inventive example under condition i as well as under condition ii was still free flowing.

table 2

|  | BASF | Aldrich | Alfa Aeser | TCI |
|---|---|---|---|---|
| 4,4'-DCDPS | 99.978 wt% | 99.92 wt% | 99.82 wt% | 99.69 wt% |
| DCDPS Isomers* | 90 ppm | 170 ppm | 370 ppm | 40 ppm |
| DCDPSO** | 0 | 40 ppm | 190 ppm | 780 ppm |
| Methanol | 120 ppm | 0 | 0 | 0 |
| Monochlorbenzene | 0 | 0 | 0 | 0 |
| Toluene | 0 | 520 ppm | 690 ppm | 110 ppm |
| n-Heptanoic acid | < 20 ppm | 0 | 0 | 0 |
| * total amount of 2,4'-dichlorodiphenylsulfone and 3,4'-dichlorodiphenylsulfone<br>** total amount of 2,4'-dichlorodiphenylsulfoxide and 4,4'- dichlorodiphenylsulfoxide<br>0 means not detectable via GC<br>ppm refer to weight ppm w<br>missing amounts to 100 wt% are other impurities | | | | |

Moreover, samples of 4,4'-dichlorodiphenylsulfone (4,4'-DCDPS) were obtained from the commercial suppliers Sigmar Aldrich, Alfa Aeser and TCI. The compositions of the commercial available 4,4'-dichlorodiphenylsulfone samples are given above in table 2. The bulk density, the tapered density, the Hausner ratio and the flowability according to Jenike for the commercial samples are given below in table 3.

Table 3

| Supplier | Bulk density kg/m3 | Tapered density 1250 kg/m3 | Hausner ratio 1250 | ffc |
|---|---|---|---|---|
| Aldrich | 684 | 838 | 1,23 | 11 |
| TCI | 837 | 974 | 1,16 | 242 |
| Alfa Aesar | 579 | 759 | 1,31 | 6 |

Example 2 of CN 106588719 was repeated. The purity of the obtained 4,4'-dichlorodiphenylsulfone was determined via GC analysis as described above. The purity was 99.69 wt%.

As can be seen from the examples above, the crystal composition (CC) according to the invention show high purity combined with a low bulk density and a good flowability. Moreover, the crystal composition (CC) according to the invention has a good storability.

The 4,4'-dichlorodiphenylsulfone compositions known in the state of the art show a higher amount of impurities as well as a higher amount solvents. To improve the purity of the commercial 4,4'-dichlorodiphenylsulfone samples these samples are dissolved in acetone and recrystallized. The recrystallization from acetone leads to a higher purity. However, after the recrystallization of the commercial samples they show a higher bulk density and a poor flowability.

Figures

Figure 1A    illustrates the measurement of $X_{c\,min}$

Figure 1B    illustrates the measurement of $X_{Fe\,max}$

**Claims**

1. Crystal composition (CC) comprising crystals (C), wherein the crystals (C) consist of

    (a) at least 99.95 % by weight of 4,4'-dichlorodiphenylsulfone,
    (b) 0 to 0.05 % by weight of impurities, and
    (c) 0 to 0.05% by weight of at least one solvent,

    based on the total weight of the crystals (C) contained in the crystal composition (CC), wherein the crystal composition (CC) has a bulk density determined according to EN ISO 60:2000-01 in the range of 570 to 750 kg/m$^3$.

2. The crystal composition (CC) according to claim 1, wherein the crystal composition (CC) comprises at least 95 % by weight of crystals (C), based on the total weight of the crystal composition (CC).

3. The crystal composition (CC) according to claim 1 or 2, wherein the tapered density determined according to DIN ISO 787 part 11 of the crystal composition (CC) is in the range of 750 to 850 kg/m$^3$.

4. The crystal composition (CC) according to any of claims 1 to 3, wherein the Hausner ratio of the crystal composition (CC) is in the range of 1.05 to 1.25.

5. The crystal composition (CC) according to any of claims 1 to 4, wherein the flowability (ff$_c$) according to Jenike of the crystal composition (CC) is in the range of 10 to 50.

6. The crystal composition (CC) according to any of claims 1 to 5, wherein the average aspect ratio of the crystals (C) contained in the crystal composition (CC) is in the range of 0.2 to 1.

7. The crystal composition (CC) according to any of claims 1 to 6, wherein the average sphericity (SPHT$_3$) of the crystals (C) contained in the crystal composition (CC) is in the range of 0.6 to 0.9.

8. The crystal composition (CC) according to any of claims 1 to 7, wherein the crystal composition (CC) has

    a d10x$_{c\,min}$-value in the range of 30 to 120 $\mu$m,
    a d50x$_{c\,min}$-value in the range of 150 to 350 $\mu$m and
    a d90x$_{c\,min}$-value in the range of 300 to 600 $\mu$m.

9. The crystal composition (CC) according to any of claims 1 to 8 has an APHA-color number determined according to ASTM D1209 in the range of 0 to 50.

10. The crystal composition (CC) according to any of claims 1 to 9, wherein the impurities (b) comprise at least 90 % by weight of one or more compounds selected from the group consisting of 2,4'-dichlorodiphenylsulfone, 3,4'-dichlorodiphenylsulfone, 4,4'-dichlorodiphenylsulfoxide, 2,4'-dichlorodiphenylsulfoxide and one or more carboxylic acid compound(s), in each case based on the total weight of the impurities (b) contained in the crystals (C).

11. The crystal composition (CC) according to any of claims 1 to 10, wherein the overall content of the isomers 2,4'-dichlorodiphenylsulfone and 3,4'-dichlorodiphenylsulfone, in the crystals (C) is in the range of 0 to 300 ppm by weight, based on the total weight of the crystals (C).

**12.** The crystal composition (CC) according to any of claims 1 to 11, wherein the content of monochlorobenzene in the crystals (C) is in the range of 0 to 50 ppm by weight, in each case based on the total weight of the crystals (C).

**13.** The crystal composition (CC) according to any of claims 1 to 12, wherein the content of toluene in the crystals (C) is in the range of 0 to 50 ppm by weight, in each case based on the total weight of the crystals (C).

**14.** The crystal composition (CC) according to any of claims 1 to 13, wherein the overall content of the isomers 4,4'-dichlorodiphenylsulfoxide and, 2,4'-dichlorodiphenylsulfoxide, in the crystals (C) is in the range of 0 to 50 ppm by weight, based on the total weight of the crystals (C).

**15.** The crystal composition (CC) according to any of claims 1 to 14, wherein the overall content of the carboxylic acid compound(s) in the crystals (C) is in the range of 0 to 200 ppm by weight, based on the total weight of the crystals (C).

**Patentansprüche**

**1.** Kristallzusammensetzung (CC), umfassend Kristalle (C), wobei die Kristalle (C) bestehen aus:

(a) mindestens 99,95 Gew.% 4,4'-Dichlordiphenylsulfon,
(b) 0 bis 0,05 Gew.% Verunreinigungen und
(c) 0 bis 0,05 Gew.% von mindestens einem Lösungsmittel,

bezogen auf das Gesamtgewicht der in der Kristallzusammensetzung (CC) enthaltenen Kristalle (C), wobei die Kristallzusammensetzung (CC) eine gemäß EN ISO 60:2000-01 bestimmte Schüttdichte im Bereich von 570 bis 750 kg/m$^3$ hat.

**2.** Kristallzusammensetzung (CC) nach Anspruch 1, wobei die Kristallzusammensetzung (CC) mindestens 95 Gew.% Kristalle (C) umfasst, bezogen auf das Gesamtgewicht der Kristallzusammensetzung (CC).

**3.** Kristallzusammensetzung (CC) nach Anspruch 1 oder 2, wobei die gemäß DIN ISO 787, Teil 11, bestimmte Stampfdichte der Kristallzusammensetzung (CC) im Bereich von 750 bis 850 kg/m$^3$ liegt.

**4.** Kristallzusammensetzung (CC) nach einem der Ansprüche 1 bis 3, wobei das Hausner-Verhältnis der Kristallzusammensetzung (CC) im Bereich von 1,05 bis 1,25 liegt.

**5.** Kristallzusammensetzung (CC) nach einem der Ansprüche 1 bis 4, wobei die Fließfähigkeit (ff$_c$) gemäß Jenike von der Kristallzusammensetzung (CC) im Bereich von 10 bis 50 liegt.

**6.** Kristallzusammensetzung (CC) nach einem der Ansprüche 1 bis 5, wobei das durchschnittliche Aspektverhältnis der in der Kristallzusammensetzung (CC) enthaltenen Kristalle (C) im Bereich von 0,2 bis 1 liegt.

**7.** Kristallzusammensetzung (CC) nach einem der Ansprüche 1 bis 6, wobei die durchschnittliche Sphärizität (SPHT$_3$) der in der Kristallzusammensetzung (CC) enthaltenen Kristalle (C) im Bereich von 0,6 bis 0,9 liegt.

**8.** Kristallzusammensetzung (CC) nach einem der Ansprüche 1 bis 7, wobei die Kristallzusammensetzung (CC)

einen d10x$_{c\,min}$-Wert im Bereich von 30 bis 120 $\mu$m,
einen d50x$_{c\,min}$-Wert im Bereich von 150 bis 350 $\mu$m und
einen d90x$_{c\,min}$-Wert im Bereich von 300 bis 600 $\mu$m hat.

**9.** Kristallzusammensetzung (CC) nach einem der Ansprüche 1 bis 8, die eine gemäß ASTM D1209 bestimmte APHA-Farbzahl im Bereich von 0 bis 50 hat.

**10.** Kristallzusammensetzung (CC) nach einem der Ansprüche 1 bis 9, wobei die Verunreinigungen (b) mindestens 90 Gew.% von einer oder mehreren Verbindungen ausgewählt aus der Gruppe bestehend aus 2,4'-Dichlordiphenylsulfon, 3,4'-Dichlordiphenylsulfon, 4,4'-Dichlordiphenylsulfoxid, 2,4'-Dichlordiphenylsulfoxid und einer oder mehreren Car-bonsäureverbindung(en) umfassen, in jedem Fall bezogen auf das Gesamtgewicht der in den Kristallen (C) enthaltenen Verunreinigungen (b).

**11.** Kristallzusammensetzung (CC) nach einem der Ansprüche 1 bis 10, wobei der Gesamtgehalt der Isomere 2,4'-Dichlordiphenylsulfon und 3,4'-Dichlordiphenylsulfon in den Kristallen (C) im Bereich von 0 bis 300 Gew.ppm liegt, in jedem Fall bezogen auf das Gesamtgewicht der Kristalle (C).

**12.** Kristallzusammensetzung (CC) nach einem der Ansprüche 1 bis 11, wobei der Gehalt an Monochlorbenzol in den Kristallen (C) im Bereich von 0 bis 50 Gew.ppm liegt, in jedem Fall bezogen auf das Gesamtgewicht der Kristalle (C).

**13.** Kristallzusammensetzung (CC) nach einem der Ansprüche 1 bis 12, wobei der Gehalt an Toluol in den Kristallen (C) im Bereich von 0 bis 50 Gew.ppm liegt, in jedem Fall bezogen auf das Gesamtgewicht der Kristalle (C).

**14.** Kristallzusammensetzung (CC) nach einem der Ansprüche 1 bis 13, wobei der Gesamtgehalt der Isomere 4,4'-Dichlordiphenylsulfoxid und 2,4'-Dichlordiphenylsulfoxid in den Kristallen (C) im Bereich von 0 bis 50 Gew.ppm liegt, in jedem Fall bezogen auf das Gesamtgewicht der Kristalle (C).

**15.** Kristallzusammensetzung (CC) nach einem der Ansprüche 1 bis 14, wobei der Gesamtgehalt der Carbonsäureverbindung(en) in den Kristallen (C) im Bereich von 0 bis 200 Gew.ppm liegt, in jedem Fall bezogen auf das Gesamtgewicht der Kristalle (C).

**Revendications**

**1.** Composition de cristaux (CC) comprenant des cristaux (C), les cristaux (C) étant constitués de

(a) au moins 99,95 % en poids de 4,4'-dichlorodiphénylsulfone,
(b) 0 à 0,05 % en poids d'impuretés, et
(c) 0 à 0,05 % en poids d'au moins un solvant, sur la base du poids total des cristaux (C) contenus dans la composition de cristaux (CC), la composition de cristaux (CC) possédant une densité en vrac déterminée selon la norme EN ISO 60:2000-01 dans la plage de 570 à 750 kg/m$^3$.

**2.** Composition de cristaux (CC) selon la revendication 1, la composition de cristaux (CC) comprenant au moins 95 % en poids de cristaux (C), sur la base du poids total de la composition de cristaux (CC).

**3.** Composition de cristaux (CC) selon la revendication 1 ou 2, la densité tassée déterminée selon la norme DIN ISO 787 partie 11 de la composition de cristaux (CC) étant dans la plage de 750 à 850 kg/m$^3$.

**4.** Composition de cristaux (CC) selon l'une quelconque des revendications 1 à 3, le rapport Hausner de la composition de cristaux (CC) étant dans la plage de 1,05 à 1,25.

**5.** Composition de cristaux (CC) selon l'une quelconque des revendications 1 à 4, la fluidité ($ff_c$) selon Jenike de la composition de cristaux (CC) étant dans la plage de 10 à 50.

**6.** Composition de cristaux (CC) selon l'une quelconque des revendications 1 à 5, le rapport d'aspect moyen des cristaux (C) contenus dans la composition de cristaux (CC) étant dans la plage de 0,2 à 1.

**7.** Composition de cristaux (CC) selon l'une quelconque des revendications 1 à 6, la sphéricité moyenne ($SPHT_3$) des cristaux (C) contenus dans la composition de cristaux (CC) étant dans la plage de 0,6 à 0,9.

**8.** Composition de cristaux (CC) selon l'une quelconque des revendications 1 à 7, la composition de cristaux (CC) possédant

une valeur de $d10x_{c\ min}$ dans la plage de 30 à 120 $\mu$m,
une valeur de $d50x_{c\ min}$ dans la plage de 150 à 350 $\mu$m et
une valeur de $d90x_{c\ min}$ dans la plage de 300 à 600 $\mu$m.

**9.** Composition de cristaux (CC) selon l'une quelconque des revendications 1 à 8 possédant un indice de couleur APHA déterminé selon la norme ASTM D1209 dans la plage de 0 à 50.

**10.** Composition de cristaux (CC) selon l'une quelconque des revendications 1 à 9, les impuretés (b) comprenant au moins 90 % en poids d'un ou plusieurs composés choisis dans le groupe constitué par la 2,4'-dichlorodiphénylsulfone, la 3,4'-dichlorodiphénylsulfone, le 4,4'-dichlorodiphénylsulfoxyde, le 2,4'-dichlorodiphénylsulfoxyde et un ou plusieurs composés de type acide carboxylique, en chaque cas sur la base du poids total des impuretés (b) contenues dans les cristaux (C).

11. Composition de cristaux (CC) selon l'une quelconque des revendications 1 à 10, la teneur globale des isomères 2,4'-dichlorodiphénylsulfone et 3,4'-dichlorodiphénylsulfone, dans les cristaux (C) étant dans la plage de 0 à 300 ppm en poids, sur la base du poids total des cristaux (C).

12. Composition de cristaux (CC) selon l'une quelconque des revendications 1 à 11, la teneur en monochlorobenzène dans les cristaux (C) étant dans la plage de 0 à 50 ppm en poids, en chaque cas sur la base du poids total des cristaux (C).

13. Composition de cristaux (CC) selon l'une quelconque des revendications 1 à 12, la teneur en toluène dans les cristaux (C) étant dans la plage de 0 à 50 ppm en poids, en chaque cas sur la base du poids total des cristaux (C).

14. Composition de cristaux (CC) selon l'une quelconque des revendications 1 à 13, la teneur globale des isomères 4,4'-dichlorodiphénylsulfoxyde et 2,4'-dichlorodiphénylsulfoxyde, dans les cristaux (C) étant dans la plage de 0 à 50 ppm en poids, sur la base du poids total des cristaux (C).

15. Composition de cristaux (CC) selon l'une quelconque des revendications 1 à 14, la teneur globale du ou des composés de type acide carboxylique dans les cristaux (C) étant dans la plage de 0 à 200 ppm en poids, sur la base du poids total des cristaux (C).

$X_{c\ min}$

$x_C$

**Fig. 1A**

$X_{Fe\ max}$

$x_{Fe}$

**Fig. 1B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018007481 A

- CN 106588719

**Non-patent literature cited in the description**

- *CHEMICAL ABSTRACTS,* 80-07-9
- **SUN, X. et al.** Iron(III) chloride (FeCl3)-catalyzed electrophilic aromatic substitution of chlorobenzene with thionyl chloride (SOCl2) and the accompanying auto-redox in sulfur to give diaryl sulfides (Ar2S): Comparison to catalysis by aluminum chloride (AlCl3). *phosphorus, sulfur, and silicon,* 2017, vol. 192 (3), 376-380

- **SUN, X. et al.** Investigations on the Lewis-acids-catalysed electrophilic aromatic substitution reactions of thionyl chloride and selenyl chloride, the substituent effects, and the reaction mechanisms. *Journal of Chemical Research,* 2013, 736-744
- CAMSIZER® Characteristics, Basics of definition DIN 66141. *Retsch Technology,* 05 November 2009